# DEMANDE DE BREVET EUROPEEN

(11) **EP 2 100 549 A1**
(43) Date de publication de la demande: **16.09.2009**
(21) Numéro de dépôt: 09154788.5
(22) Date de dépôt: 10.03.2009
(51) Int. Cl.: A61B 1/005, A61B 1/267

(54) **Endoscope électronique amélioré**

(30) Priorité: 13.03.2008 FR 0851639
(71) Demandeur: Optomed, 91974 Courtaboeuf Cedex (FR)
(72) Inventeur: Duchene, Arnaud, 91160, Longjumeau (FR)
(74) Mandataire: Blot, Philippe Robert Emile

(57) **Abrégé**

L'invention concerne un endoscope électronique (2) du type comportant : une extrémité distale (14) comportant un moyen d'éclairage et un moyen électronique d'acquisition d'images ; une sonde (12) dont l'une des extrémités est connectée à l'extrémité distale ; et, des moyens de commande (10) connectés à l'autre extrémité de la sonde, caractérisé en ce que la sonde comporte une portion déformable plastiquement (20) apte à conserver, sous l'effet de son propre poids lors de l'utilisation de l'endoscope, la forme qui lui a été conférée par un opérateur, ladite portion déformable portant l'extrémité distale de l'endoscope

## Description

La présente invention concerne un endoscope électronique du type comportant : une extrémité distale comportant un moyen d'éclairage et un moyen électronique d'acquisition d'images ; une sonde dont l'une des extrémités est connectée à l'extrémité distale ; et, des moyens de commande connectés à l'autre extrémité de la sonde.

Certaines pathologies respiratoires du larynx ou du pharynx d'un équidé, et en particulier d'un cheval de course, ne peuvent être diagnostiquées que par un examen endoscopique réalisé au cours d'un test dynamique, en utilisant un endoscope, de préférence un endoscope électronique.

Selon l'art antérieur, le moyen de commande d'un endoscope électronique comporte une source de lumière produisant un flux lumineux qui est conduit, au moyen d'une fibre optique, le long de la sonde jusqu'à l'extrémité distale. Le moyen d'éclairage de l'extrémité distale comporte des lentilles permettant de distribuer le flux lumineux reçu de la fibre optique sur la zone à examiner. Or, la source de lumière des endoscopes électroniques connus est volumineuse et nécessite un générateur de courant électrique de forte puissance, également volumineux. En conséquence, les endoscopes électroniques selon l'art antérieur sont des appareillages fixes.

C'est la raison pour laquelle le test dynamique est réalisé dans un local dédié, équipé d'une installation d'endoscopie comportant un endoscopie électronique fixe et un tapis roulant spécifique sur lequel est placé le cheval de course devant subir l'examen.

On utilise un endoscope électronique souple dont la sonde est molle et sans maintien propre. Cette caractéristique mécanique de la sonde est utilisée pour positionner l'extrémité distale. Plus précisément, l'extrémité distale est introduite par les nasaux, puis poussée dans les sinus de l'animal, de manière à être placée dans la gorge de l'animal, en vis-à-vis de la zone à examiner. La sonde qui porte l'extrémité distale prend alors appui sur les parois des sinus et de la cavité à inspecter.

Mais, dans cet environnement de test, l'animal n'est pas réellement dans des conditions normales d'entraînement. Le traumatisme d'être placé sur un tapis roulant biaise le comportement de l'animal, ce qui réduit d'autant l'intérêt d'un tel examen.

Par ailleurs, une installation d'endoscopie comportant un tapis roulant est extrêmement coûteuse. De plus, puisque cette installation est fixe, il faut transporter le cheval de course jusque dans le local d'examen, ce qui représente un coût additionnel lorsque ce local est éloigné du centre d'entraînement.

Enfin, l'image obtenue n'est pas d'une grande qualité. En effet, l'extrémité distale portée par la sonde molle et comportant le moyen électronique d'acquisition d'images se déplace par rapport à la zone à examiner au cours du test dynamique et des mouvements de l'animal. Dans des cas extrêmes, comme un mouvement brusque de la tête de l'animal, il peut se faire que la portion distale vienne en contact des muqueuses.

L'invention a donc pour but d'apporter une solution aux problèmes précités en proposant un endoscope électronique permettant de réaliser une endoscopie dans des conditions normales, par exemple d'entraînement d'un cheval de course, tout en garantissant une bonne qualité d'image.

Pour cela l'invention porte sur un endoscope électronique du type précité, caractérisé en ce que la sonde comporte une portion déformable plastiquement apte à conserver, sous l'effet de son propre poids lors de l'utilisation de l'endoscope, la forme qui lui a été conférée par un opérateur, la portion déformable portant l'extrémité distale de l'endoscope.

Suivant des modes particuliers de l'invention, l'endoscope électronique comporte une ou plusieurs des caractéristiques suivantes, prisent isolément ou suivant toutes les combinaisons techniquement possibles :
- le moyen d'éclairage comportent au moins une LED.
- le moyen de commande comporte une batterie en tant que moyen générateur de courant électrique apte à alimenter l'endoscope en puissance électrique, l'endoscope étant ainsi rendu autonome.
- la portion déformable comporte au moins une tige réalisée dans un matériau malléable adapté et dont une section transversale possède des dimensions adaptées, de sorte que la tige confère à la portion déformable la plasticité souhaitée.
- la portion déformable comporte deux tiges réalisées dans un matériau conducteur du courant électrique, et les deux tiges permettent de connecter électriquement la batterie au moyen d'éclairage de l'extrémité distale.
- le moyen de commande comporte un moyen d'émission et de transmission d'ondes radio pour communiquer avec un moyen d'émission et de transmission d'ondes radio correspondant placé à distance.
- le moyen de commande comporte un moyen de traitement permettant de traiter en temps réel les signaux générés par le moyen électronique d'acquisition d'images pour produire des images, les images produites pouvant être affichées en temps réel sur un moyen d'affichage connecté au moyen d'émission et de transmission d'ondes radio correspondant placé à distance.
- l'endoscope est adapté pour la réalisation d'un examen endoscopique sur un cheval de course au cours d'une séance d'entraînement normale de ce dernier.
- la tige est réalisée dans un alliage de cuivre, de préférence comportant 90% en poids de cuivre, et possède un diamètre compris entre 1,7 et 2,5 mm, et de préférence entre 1,75 et 2,25 mm.
- l'endoscope comporte un licol muni d'un moyen de fixation de la sonde, le moyen de fixation comportant de préférence au moins une agrafe autorisant la fixation de la sonde par clipsage.

L'invention et ses avantages seront mieux compris à la lecture de la description qui va suivre, donnée uniquement à titre d'exemple, et faite en se référant aux dessins annexés, dans lesquels :
- la figure 1 est une représentation schématique de l'endoscope électronique selon l'invention en position d'examen sur un cheval de course ;
- la figure 2 est une vue de face de l'agrafe des moyens de fixation de l'endoscope de la figure 1 ;
- la figure 3 est une section transversale de la portion déformable de l'endoscope de la figure 1 ; et,
- la figure 4 est une coupe axiale de la tête de l'extrémité distale de l'endoscope de la figure 1.

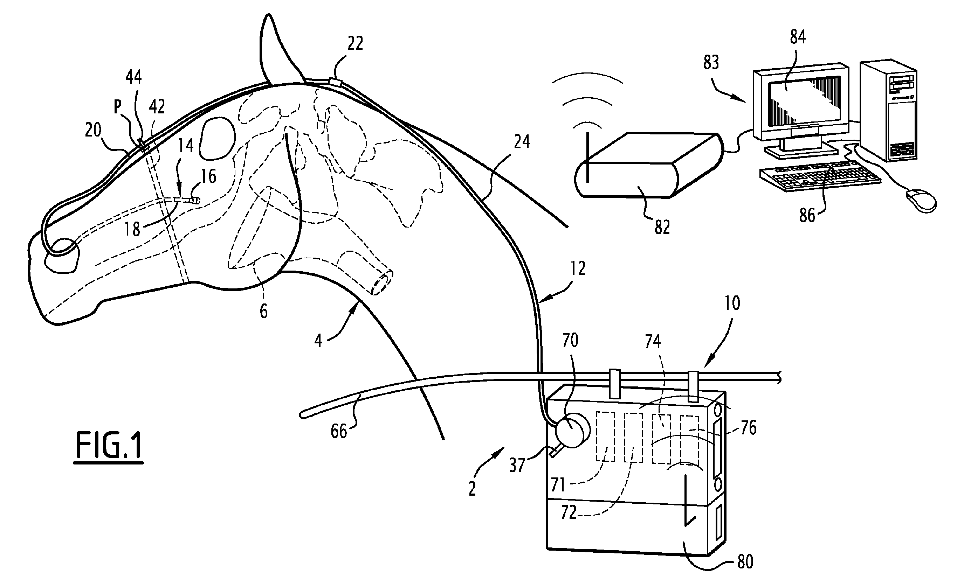

La figure 1 illustre de manière schématique l'endoscope électronique 2 selon l'invention. L'endoscope électronique 2 est représenté en position sur la tête d'un cheval 4 dont on souhaite examiner le larynx 6 au cours d'un test dynamique réalisé dans les conditions normales d'une séance d'entraînement.

L'endoscope électronique 2 comporte des moyens de commande 10, une sonde 12, et une extrémité distale 14. L'extrémité distale 14 est connectée, mécaniquement et électriquement, au moyen de commande 10 par l'intermédiaire de la sonde 12.

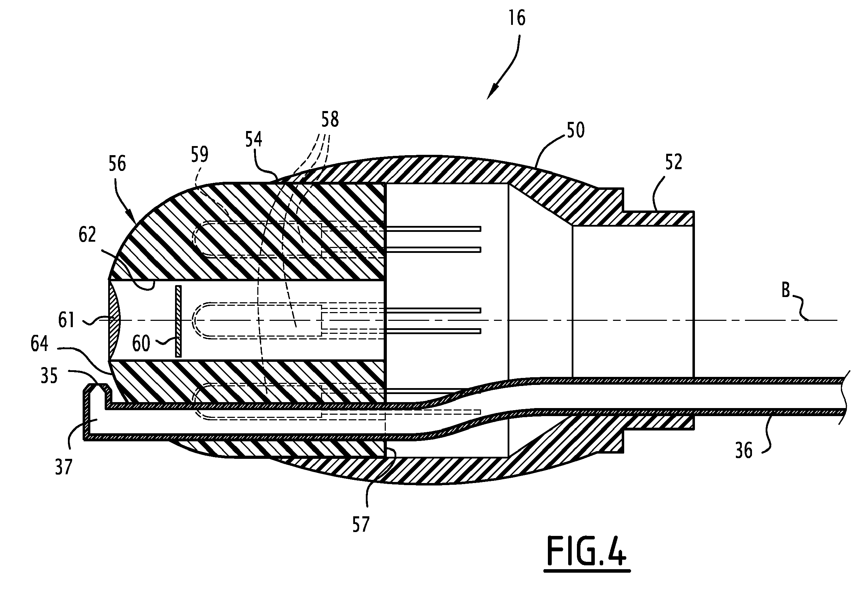

L'extrémité distale 14 comporte une tête 16 et une portion articulée 18. La tête 16 comporte un moyen d'éclairage de la cavité à examiner, ainsi qu'un moyen électronique d'acquisition d'images apte à générer un signal vidéo électrique. Comme cela sera décrit en détail lors de la description de la tête 16 en référence à la figure 4, le moyen d'éclairage comporte plusieurs diodes électroluminescentes ou LED et le moyen électronique d'acquisition d'images comporte un capteur à transfert de charges ou capteur CCD.

La portion articulée 18, fixée à l'arrière de la tête 16, relie la tête 16 à l'une des extrémités de la sonde 12. La portion articulée 18 comporte des premiers et seconds moyens de déplacement permettant d'orienter mécaniquement la tête 16 selon deux directions orthogonales entre elles.

Les moyens de commande 10 vont maintenant être décrits en détail.

Puisque le moyen d'éclairage de l'endoscope électronique 2 selon l'invention est constitué de LED, les moyens de commande 10 ne comportent pas de source de lumière, contrairement à l'art antérieur. De plus les LED consomment une puissance électrique réduite, de sorte que les moyens de commande 10 de l'endoscope 2 comportent un générateur de courant électrique qui est une batterie 72 ayant des dimensions réduites. En conséquence, les moyens de commande 10 sont compacts et sont transportables. Ils sont portés par le sujet à examiner. Dans le cas d'un cheval de course, les moyens de commande 10 peuvent être placés dans la selle, dans un sac à dos porté par le cavalier ou encore, comme cela est représenté sur la figure 1, fixé à un bras 66 du sulky tiré par le cheval 4. L'endoscope électronique selon l'invention pèse moins de 5kg.

Les moyens de commande 10 comportent un boîtier de connexion 70 mécanique et électrique de la sonde 12 ; des premier et second moyens d'entraînement 71 pour actionner respectivement les premier et second moyens de déplacement de la portion articulée 18 ; la batterie 72 d'alimentation en courant électrique de l'endoscope 2 ; un processeur 74 apte, entre autre, à traiter le signal vidéo transmis depuis le moyen électronique d'acquisition d'images ; et un moyen de mémorisation 76 permettant d'enregistrer en temps réel les images décodées par le processeur 74.

Les moyens de commande 10 comportent également un moyen d'émission et de réception 80 de signaux radio. Le moyen d'émission et de réception 80 est destiné à communiquer avec un moyen d'émission et de transmission correspondant 82 situé « au sol » et à distance. Le moyen d'émission et de transmission correspondant 82 est par exemple relié à un ordinateur 83 possédant un écran 84 et à un clavier 86 et constituant une interface de commande et de contrôle pour l'opérateur. En particulier, l'opérateur visualise en temps réel sur l'écran 84 l'image acquise par l'endoscope électronique 2, tout au long de la mise en place de l'endoscope 2 et au cours du test dynamique.

Les premier et second moyens d'entraînement pour actionner respectivement les premier et second moyens de déplacement de la portion articulée 18 peuvent prendre la forme de première et seconde molettes sur le boîtier de commande 70. Ces première et seconde molettes sont utilisées par l'opérateur qui oriente convenablement la tête 16 de l'endoscope dans une phase initiale de mise en place de l'endoscope, avant de réaliser le test dynamique. De préférence, les premier et second moyens d'entraînement sont des actionneurs 71 aptes à être commandés à distance par l'opérateur au moyen de l'ordinateur 83 et des moyens de communication radio 80 et 82.

La sonde 12 est connectée, à l'une de ses extrémités, à la portion articulée 18 de l'extrémité distale 14, et est reliée, à l'autre extrémité, au boîtier de connexion 70 des moyens de commande 10. La sonde 12 a pour fonction de connecter électriquement les moyens de commande 10 au moyen d'éclairage et au moyen d'acquisition d'images de la tête 16, et de connecter mécaniquement les moyens de commande 10 à la portion articulée 18.

De plus, la sonde 12 de l'endoscope électronique 2 selon l'invention a pour fonction de maintenir l'extrémité distale 14 dans une position prédéfinie à l'intérieur de la cavité à examiner. Pour cela, la sonde 14 comporte, successivement selon sa longueur, une portion déformable 20, un élément de raccord 22 et une portion passive 24. La portion déformable 20 s'étend entre la portion articulée 18 de l'extrémité distale 14 et le moyen de raccord 22. La portion passive 24 s'étend entre le raccord 22 et le boîtier de connexion 70.

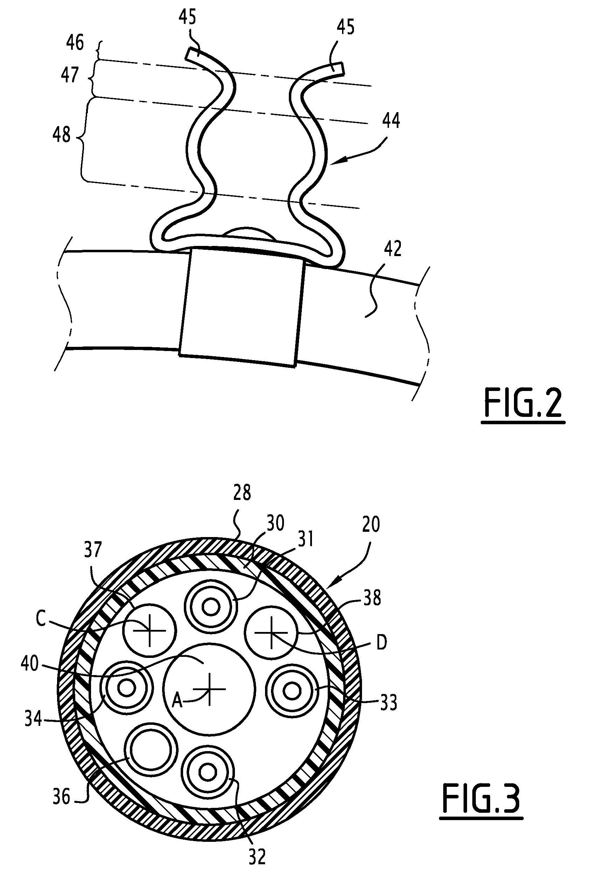

La portion déformable 20 de la sonde 14 va maintenant être décrite en détail par référence à la figure 3 qui en présente une section transversale. La portion déformable 20 est localement de forme cylindrique autour d'un axe A. Elle a un diamètre inférieur à 1 cm. Elle est délimitée extérieurement par une gaine tubulaire 28 en un matériau plastique, tel que par exemple du PVC. La face orientée radialement vers l'intérieur de la gaine 28 est renforcée par une lame métallique spirée 30. Celle-ci constitue une armature de la portion déformable 20 autorisant les déformations par courbure dans un plan axial.

A l'intérieur du volume délimité par la lame métallique spirée 30, la portion déformable 20 de la sonde 12 comporte une première paire de câbles 31 et 32 destinés à être couplés, d'une part, aux premiers moyens de déplacement mécaniques de la portion articulée 18 et, d'autre part, au premier moyen d'entraînement des moyens de commande 10 pour orienter la tête 16 selon la première direction. La portion déformable 20 comporte également une seconde paire de câbles 33 et 34 destinés à être couplés, d'une part, aux seconds moyens mécaniques de la portion articulée 18 et, d'autre part, au second moyen d'entraînement des moyens de commande 10 pour orienter la tête 16 selon la seconde direction.

La sonde 14 comporte également un canal d'irrigation 36 permettant d'amener de l'air et/ou de l'eau depuis un orifice d'entrée 37 situé sur le boîtier de connexion 70, vers un orifice de sortie 35 situé sur la tête 16 de l'extrémité distale 14. Le canal d'irrigation 36 s'étend donc à travers la portion passive 24, le raccord 22, la portion déformable 20, la portion articulée 18 et la tête 16.

La portion déformable 20 de la sonde 12 comporte au moins une tige déformable élastiquement. Dans le mode de réalisation décrit, la portion déformable 20 comporte une première tige 37 et une seconde tige 38. Les première et seconde tiges 37 et 38 sont cylindriques et leurs axes respectifs C et D sont disposés parallèlement à l'axe A. Les première et seconde tiges 37 et 38 ont pour fonction de conférer à la portion déformable 20 la caractéristique mécanique d'être à la fois déformable plastiquement et rigide : plus précisément, la portion déformable 20 est déformable plastiquement car elle est apte à être déformée lors de l'application de contraintes raisonnables, c'est-à-dire de contraintes pouvant être générées par la force musculaire d'un opérateur, puis à conserver la forme ainsi donnée par l'opérateur alors que la contrainte de déformation a cessé d'être appliquée ; la portion déformable 20 est rigide au sens où elle conserve la forme donnée par l'opérateur au cours de l'utilisation de l'endoscope, c'est-à-dire qu'elle présente une rigidité suffisante par rapport aux contraintes générées, lors du test dynamique, par le propre poids et les accélérations de la portion déformable 20.

De préférence, la première tige 37, respectivement la seconde tige 38, est réalisée dans un matériau malléable.

Les première et seconde tiges 37 et 38 ont également pour fonction de conduire le courant électrique depuis la batterie 72 vers le moyen d'éclairage de la tête 16. De préférence, la première tige 37, respectivement la seconde tige 38, est alors réalisée dans un matériau conducteur du courant électrique.

Dans le mode de réalisation actuellement préféré, les première et seconde tiges 37 et 38 sont en un matériau métallique, de préférence en un alliage de cuivre.

Pour un endoscope électronique selon l'invention adapté à la réalisation d'un examen endoscopique sur un cheval de course, la portion déformable 20 est placée sur la tête de l'animal de manière à porter à faux, au-delà d'un point de fixation P, sur une longueur d'environ 1 mètre. Les première et seconde tiges 37 et 38 sont réalisées dans un alliage de 90% en poids de cuivre et présentent un diamètre entre 1,5 et 2,5 et 2,5 mm, et de préférence entre 1,75 et 2,25 mm. Les première et seconde tiges 37 et 38 n'ont pas à avoir un même diamètre.

On notera que dans la portion passive 24 de la sonde 12, portion qui est flexible sans conservation de sa forme, du premier et second fils électriques normaux sont substitués aux premières et secondes tiges 37 et 38 de la portion déformable 20. Les premier et second fils électriques sont connectés électriquement, d'une part, aux première et seconde tiges 37 et 38, et, d'autre part, à la batterie 72, via le boîtier de connexion 70.

Enfin, la portion déformable 20 comporte un câble vidéo 40 disposé par exemple le long de l'axe A. Le câble vidéo 40 est destiné à transmettre le signal vidéo généré par le moyen électronique d'acquisition d'images de la tête 16 vers le moyen de traitement 74 des moyens de commande 10. Le câble vidéo 40 s'étend donc du moyen électronique d'acquisition d'images jusqu'au boîtier de connexion 70.

L'endoscope électronique 2 comporte des moyens de fixation sur le sujet à examiner. Dans le cas d'un cheval de course, la sonde 12 est fixée sur un licol 42 spécifique, comportant une agrafe 44 représentée en détail à la figure 2. L'agrafe 44 en acier est rivetée sur le licol 42 en cuire. L'agrafe 44 est conformée de manière à ce que la sonde 12 soit fixée par clipsage. Plus précisément, l'agrafe 44 comporte deux bras latéraux 45. Dans une section supérieure de l'agrafe 44, les deux bras latéraux 45 s'écartent l'un de l'autre de manière à munir l'agrafe 44 d'une section de guidage 46 en forme de « V » destinée à aider le guidage de la sonde 12 lors de sa fixation. Puis, dans une section intermédiaire, les deux bras 45 sont proches l'un de l'autre de manière à former une section d'étranglement 47 dont la largeur est inférieure au diamètre de la sonde 12. Enfin, dans une section inférieure, les deux bras 45 sont conformés en arcs de cercle dont le diamètre correspond à celui de la sonde 12, de manière à définir une section de maintien 48 de la sonde. La sonde est mise en butée contre les deux bras latéraux 45 au niveau de la section d'étranglement 46. L'opérateur insère la sonde 12 à force entre les deux bras latéraux 45 qui s'écartent élastiquement l'un de l'autre, jusqu'à ce que la sonde 12 puisse franchir la section d'étranglement 47 et venir se loger dans la section de maintien 48. Une fois en position dans la section de maintien 48, la sonde 12 peut tourner autour de son axe A de manière à ce que l'opérateur puisse par exemple aligner la première direction de déplacement de la tête 16 de l'endoscope 2 selon la direction verticale.

En se référant maintenant à la figure 4, la tête 16 de l'endoscope électronique 2 va être décrite en détail. On observera qu'une telle tête d'endoscope peut être réutilisée indépendamment des caractéristiques précitées de l'endoscope. La tête 16 comporte un carter 50 de forme cylindrique autour d'un axe B. Le bord arrière 52 du carter 50 comporte un épaulement sur lequel vient prendre appui la portion articulée 18 tubulaire. Le bord avant 54 du carter 50 porte une paroi translucide 56 obturant l'avant du carter 50. Elle est par exemple en plexiglas. La paroi translucide 56 est bombée de manière à former une calotte hémisphérique faisant saillie axialement au-delà du bord avant 54 du carter 50.

Dans le volume intérieur du carter 50, la tête 16 comporte un moyen d'éclairage constitué d'au moins une LED 58 et de préférence de six LED 58. Les LED 58 sont alimentées en courant électrique par la batterie 72, via le boîtier de connexion 70, les premier et second fils électriques de la portion passive 24, le raccord 22, les première et seconde tiges 37 et 38 de la portion déformable 20 de la sonde 12 et des connecteurs électriques au niveau de la portion articulée 18. Les LED 58 sont choisies pour produire un flux lumineux caractérisé par une température de 6000°, ce qui correspond à la lumière naturelle de sorte que les images obtenues au moyen de l'endoscope 2 sont d'une grande qualité. Dans le mode de réalisation décrit, six LED 58 sont disposées régulièrement le long d'un anneau d'axe B. Ainsi, l'intensité du flux lumineux produit par la tête 16 est très uniforme dans le champ conique d'éclairage. Une LED 58, de forme oblongue est disposée parallèlement à l'axe B dans un alésage 59 ménagé dans la face intérieure 57 de la paroi translucide 56. Ainsi, une LED 58 est placée en avant du bord avant 54 du carter 50 opaque. Le champ conique d'éclairage présente de ce fait une ouverture large, de l'ordre de 140°. La paroi 56 est choisie translucide pour diffuser la lumière générée par les différentes LED 58 et améliorer l'uniformité de l'éclairage produit. Le rendement du moyen d'éclairage mis en oeuvre dans la tête 16 de l'endoscope 2 selon l'invention étant très élevé, on constatera qu'il n'y a plus besoin de recourir à des lentilles pour concentrer le flux lumineux généré.

La tête 16 renferme également un moyen électronique d'acquisition d'images. Il s'agit d'un capteur CCD 60, d'un objectif 61 et de l'électronique d'acquisition de bas niveau requise pour générer un signal vidéo. Le signal vidéo généré est transmis au processeur 74 via un connecteur adapté dans la portion articulée 18, le câble vidéo 40 circulant le long de la sonde 12 et le boîtier de connexion 70. Le capteur CCD 60 est logé dans un évidement axial 62 traversant la paroi translucide 56. L'évidement axial 62 est fermé au niveau de la face extérieure 64 de la paroi translucide 56 par l'objectif 61 qui présente un dioptre extérieur plan. Le capteur CCD 60 est situé le long de l'axe B, derrière l'objectif 61, mais en avant des LED 58. Eventuellement, la paroi axiale de l'évidement 62 est opacifiée pour que le capteur CCD ne soit pas perturbé par la lumière produite par les LED 58.

Le canal d'irrigation 36 de la sonde 12 se prolonge dans l'extrémité distale 14 et traverse la paroi translucide 56. Il se termine par un embout 37 placé sur la face extérieure 64 de la paroi translucide 56. Un canal interne de l'embout 37 fait un coude à 90° par rapport à l'axe du canal d'irrigation 36 et est orienté de manière à projeter par un orifice 35, de l'air et/ou de l'eau sur l'objectif 61 du moyen d'acquisition électronique d'images.

L'endoscope électronique selon l'invention permet donc de réaliser une image endoscopique alors que le sujet se trouve dans des conditions normales.

Une fois positionné, l'endoscope électronique selon l'invention permet d'obtenir une image de bonne qualité grâce au moyen d'éclairage générant une puissance lumineuse importante et uniforme, une portion articulée permettant de déplacer le moyen d'acquisition d'images de manière à cadrer la zone à examiner.

L'extrémité distale de l'endoscope électronique est suspendue à l'intérieur de la cavité à imager mais n'oscille pas au cours du test dynamique. Il suit les mouvements de la tête du cheval et ne risque pas de rentrer en contact avec les muqueuses de la cavité inspectée. La portion déformable peut être conformée par l'opérateur de manière à suivre la courbure anatomique des canaux empruntés pour accéder à la cavité à inspecter.

L'homme du métier constatera que, l'endoscope électronique selon l'invention étant dépourvue de fibre optique, il peut être déformé de manière à adopter n'importe quelle courbure sans que cela ait une influence sur la qualité de l'image obtenue.

## Revendications

1. Endoscope électronique (2) du type comportant :
- une extrémité distale (14) comportant un moyen d'éclairage (58) et un moyen électronique d'acquisition d'images (60);
- une sonde (12) dont l'une des extrémités est connectée à l'extrémité distale ; et,
- des moyens de commande (10) connectés à l'autre extrémité de la sonde, **caractérisé en ce que** la sonde comporte une portion déformable plastiquement (20) apte à conserver, sous l'effet de son propre poids lors de l'utilisation de l'endoscope, la forme qui lui a été conférée par un opérateur, ladite portion déformable portant l'extrémité distale de l'endoscope.

2. Endoscope selon la revendication 1, **caractérisé en ce que** le moyen d'éclairage comportent au moins une LED (58).

3. Endoscope selon la revendication 2, **caractérisé en ce que** le moyen de commande (10) comporte une batterie (72) en tant que moyen générateur de courant électrique apte à alimenter ledit endoscope (2) en puissance électrique, l'endoscope étant ainsi rendu autonome.

4. Endoscope selon l'une quelconque des revendications précédentes, **caractérisé en ce que** la portion déformable (20) comporte au moins une tige (37, 38) réalisée dans un matériau malléable adapté et dont une section transversale possède des dimensions adaptées, de sorte que ladite au moins une tige confère à la portion déformable la plasticité souhaitée.

5. Endoscope selon les revendications 3 et 4 en combinaison, **caractérisé en ce que** la portion déformable (20) comporte deux dites tiges (37, 38) réalisées dans un matériau conducteur du courant électrique, et **en ce que** en lesdites deux tiges permettent de connecter électriquement la batterie (72) au moyen d'éclairage (58) de l'extrémité distale (14).

6. Endoscope selon l'une quelconque des revendications précédentes, **caractérisé en ce que** le moyen de commande (10) comporte un moyen d'émission et de transmission d'ondes radio (80) pour communiquer avec un moyen d'émission et de transmission d'ondes radio correspondant (82) placé à distance.

7. Endoscope selon la revendication 6, **caractérisé en ce que** le moyen de commande (10) comporte un moyen de traitement (74) permettant de traiter en temps réel les signaux générés par le moyen électronique d'acquisition d'images (60) pour produire des images, les images produites pouvant être affichées en temps réel sur un moyen d'affichage (84) connecté au moyen d'émission et de transmission d'ondes radio correspondant (82) placé à distance.

8. Endoscope selon l'une quelconque des revendications précédentes, **caractérisé en ce qu'**il est adapté pour la réalisation d'un examen endoscopique sur un cheval de course au cours d'une séance d'entraînement normale de ce dernier.

9. Endoscope selon la revendication 7 et la revendication 3 en combinaison, **caractérisé en ce que** ladite au moins une tige (37, 38) est réalisée dans un alliage de cuivre, de préférence comportant 90% en poids de cuivre, et possède un diamètre compris entre 1,7 et 2,5 mm, et de préférence entre 1,75 et 2,25 mm.

10. Endoscope selon la revendication 8 ou la revendication 9, **caractérisé en ce qu'**il comporte un licol (42) muni d'un moyen de fixation de la sonde, le moyen de fixation comportant de préférence au moins une agrafe (44) autorisant la fixation de la sonde par clipsage.
